(19) Europäisches Patentamt
European Patent Office
Office européen des brevets

(11) Publication number: **0 068 375**
**A2**

(12) **EUROPEAN PATENT APPLICATION**

(21) Application number: 82105405.3

(22) Date of filing: 19.06.82

(51) Int. Cl.³: C 12 N 15/00, C 07 H 21/04, C 07 C 103/52, C 12 N 1/00, C 12 P 21/02 // C12R1/19

(30) Priority: 22.06.81 GB 8119138

(43) Date of publication of application: 05.01.83 Bulletin 83/1

(84) Designated Contracting States: BE CH DE FR GB IT LI NL SE

(71) Applicant: G.D. Searle & Co., Box 1045, Skokie, Illinois 60076 (US)

(72) Inventor: Stewart, Andrew George, 4, Amersham Road, High Wycombe Buckinghamshire (GB)
Inventor: Bell, Leslie David, 13, Brookside, Thame Oxfordshire (GB)

(74) Representative: Werner, Hans-Karsten, Dr. et al, Deichmannhaus am Hauptbahnhof, D-5000 Köln 1 (DE)

(54) **Recombinant DNA techniques for the production of relaxin.**

(57) Synthetic genes for relaxin, the expression thereof, corresponding plasmid recombinants and transformed cells and the production thereof are disclosed.

Relaxin has an application in controlling labour in pregnancy.

**EP 0 068 375 A2**

This invention relates to recombinant DNA techniques for the production of relaxin; more particularly, it relates to synthetic genes for relaxin and to the expression thereof, to corresponding plasmid recombinants and transformed cells and to the production thereof.

Relaxin is a polypeptide hormone which is produced in the ovaries and certain other tissues, especially during pregnancy. Although synthesised and stored in the ovaries, serum immunoreactive relaxin rises, often dramatically, towards the end of pregnancy in pigs, rats, guinea pigs, mice and hamsters. Relaxin is known to have effects on the pubic symphysis, uterus, mammary glands and connective tissue, it causes cervical ripening and initiates parturition, (see, for example, Steinetz, B.G., et al, (1980), Dilatation of the Uterine Cervix, Eds. Naftolin, F., and Stubblefield, P.G., Raven Press, New York, 157-177; and Schwabe, C., et al, (1980), Recent Progress In Hormone Research, Relaxin, 34, 123-199).

Relaxin shows remarkable cross-species reactivity both in *in vivo* and *in vitro* tests and in its antigenicity to such an extent that porcine relaxin has been used in a clinical trial on patients requiring surgical induction of labour, (see, for example, Steinetz, B.G., et al, loc cit; Schwabe, C., et al, loc cit; O'Byrne, E.M., and Steinetz, B.G., (1976), Proc. Soc. Exp. Biol. Med., 152, 272-276; and Larkin, L.H., et al, (1979), Acta Endocrinologica, 92, 568-576). After administration of relaxin, most patients

had improved cervical scores and significantly fewer required augmentation in labour with oxytocin than the control group, no side effects were shown, (see, for example, MacLennan, A.H., et al, (1980), The Lancet, 2 February, 220-223). Porcine relaxin is therefore considered to be useful in the induction and control of labour.

Administration of porcine relaxin to sows during farrowing may also be of use in reducing the rate of still-birth of piglets. It has been observed that, especially with large litters, mortality tends to occur with the last of the litter, (see, for example, Asdell, S.A., and Willman, J.P. (1941), J. Ag. Res., 63, 345-353) when the level of relaxin may decline.

Relaxin is one of a number of insulin-like polypeptides and comprises two polypeptide chains, A and B, connected by disulphide bridges, (see, for example, Schwabe, C., and McDonald, J.K., (1977), Science, 197, 914-915; Bedarkar, S., et al, (1977), Nature, 270, 449-451; and Isaacs, N., et al, (1978), Nature, 271, 278-281). The porcine A chain comprises 22 amino acids as illustrated in Figure 1 of the accompanying drawings. Apparently, some microheterogeneity exists in that a proportion of relaxin has been found to have an A chain wherein from 10 to 20% of the glutamine at position 10 is replaced by glutamic acid, (see, for example, Niall, H.D., et al, (1980), Insulin: chemistry, structure and function of

insulin and related hormones, Eds. Brandenburg, D., and Wollmer, A., Walter de Gruyter, New York, 719-726). The porcine B chain comprises from 28 to 31 amino acids as illustrated in Figure 2 of the accompanying drawings wherein PGA represents pyroglutamic acid. The carboxy terminus of the B chain appears to be heterogeneous, which is indicated in the drawing by vertical lines. As

usually isolated, the major components of peaks termed CM-a' (used in the clinical trial) and CM-a have B chains of 29 and 31 residues respectively, (see, for example, Niall, H.D., et al, loc cit). Another reported B chain structure comprises 26 residues and corresponds to the above-mentioned structure, except that residues 24 to 26 are Val-Trp-Ser, (see, for example, Schwabe, C., et al, (1977), Biochem. Biophys. Res. Comm., 75, 503-510). Also, a B chain structure comprising 30 residues has been reported which is similar, except that residues 27 to 30 are Thr-Trp-Gly-Arg, (see, for example, Kwok, S., and Bryant-Greenwood, G., (1977), Nature, 267, 544-546).

On the basis of the known amino acid sequences illustrated in accompanying Figures 1 and 2, novel synthetic gene sequences have been determined corresponding to the A chain and the B chain separately and to a hybrid B-C-A gene wherein the C sequence codes for a peptide link between the B and A chains. It was decided to base the linking C sequence on the peptide -Asp-Pro-, which is known to be sensitive to hydrolysis at about pH 2, (see, for example, London, M., (1977), Methods in Enzymology 47, 145). The actual sequence chosen for the peptide was -Asp-Pro-Gly-Asp-Pro- (see Figure 3 of the accompanying drawings), which allows the A and B chains to assume the normal conformation without distortion. The hybrid B-C-A is produced as a single peptide, which may be used without modification or may be cleaved at low pH to

remove the C peptide.

In the selection of an optimum sequence from the large number of codon possibilities, arising from the degeneracy of the genetic code, several criteria have been observed. Firstly, trinucleotide codons should be used which are most acceptable to the cells in which the gene will be expressed, in particular E. coli, (see, for example, Grantham, R., et al, (1980), Nucl. Acids.Res., 8, r47-r62). Secondly, the 5' terminus of the gene should have a restriction site, such as an EcoRI site, such that, when the "sticky end" is treated with S1 nuclease, the "blunt end" produced will be exactly at the 5' end of the coding sequence. Thirdly, the 3' end should have a chain terminator codon. Fourthly, the 3' end should have a restriction site, such as a Hind III site, for use for insertion into an appropriate plasmid. Fifthly, the restriction sites at the 5' and 3' ends should enable the gene to be cloned into and excised from a suitable plasmid, such as pBR 322, (see, for example, Bolivar, F., et al, (1977), Gene, 2, 95-113). Sixthly, sequences within the gene which are self-complementary or are complementary to the other sequences (other than the correct sequence) on either strand or the doublestranded gene should be kept to a minimum.

Using a computer, energies arising from all possible interactions were calculated, (see, for example, Tinoco, Jr., I., et al, (1971), Nature, 230, 362-367; and

Powers, G.J., et al, (1975), JACS, 97, 875-889), so that off-diagonal interactions might be avoided as far as possible.

In one embodiment, the present invention relates to a synthetic gene for the expression of the porcine relaxin A chain which comprises the following sequence:

| | | | | | | |
|---|---|---|---|---|---|---|
| 5' | CGC | ATG | ACT | CTG | TCC | - |
| 3' | GCG | TAC | TGA | GAC | AGG | - |
| | GAG | AAA | TGT | TGC | CAG | - |
| | CTC | TTT | ACA | ACG | GTC | - |
| | GTT | GGT | TGT | ATC | CGT | - |
| | CAA | CCA | ACA | TAG | GCA | - |
| | AAA | GAC | ATT | GCT | CGT | - |
| | TTT | CTG | TAA | CGA | GCA | - |
| | CTG | TGT | 3' | | | |
| | GAC | ACA | 5' | | | |

In a further embodiment of the present invention, the A gene contains, in addition to the coding sequence, a stop codon, restriction sites for EcoRI and Hind III in order to facilitate insertion into plasmids, such as pAT 153, (see, for example, Twigg, A.J., and Sherratt, D., (1980), Nature, 283, 216-218), or expression plasmids, in particular pWT 571, (see, for example, published European Patent Application No. 52002),

a Hinf I restriction site which enables a gene lacking codons for Arg and Met to be cloned (the product for such a gene lacking Arg and with or without the terminal initiator methionine introduced

from the expression plasmid may be more active than the original gene containing the Arg residue with or without an initiator methionine) and an EcoR2 restriction site, (see Figure 4 of the accompanying drawings).

In this embodiment, the present invention relates to a synthetic gene for the expression of the porcine relaxin A chain which comprises the following sequence:

| | | | | | | | | |
|---|---|---|---|---|---|---|---|---|
| 5' | A | ATT | CGC | ATG | ACT | CTG | TCC | - |
| 3' | | | GCG | TAC | TGA | GAC | AGG | - |
| | | | GAG | AAA | TGT | TGC | CAG | - |
| | | | CTC | TTT | ACA | ACG | GTC | - |
| | | | GTT | GGT | TGT | ATC | CGT | - |
| | | | CAA | CCA | ACA | TAG | GCA | - |
| | | | AAA | GAC | ATT | GCT | CGT | - |
| | | | TTT | CTG | TAA | CGA | GCA | - |
| | | | CTG | TGT | TA | | 3' | |
| | | | GAC | ACA | ATT | CGA | 5' | |

In another embodiment, the present invention relates to a synthetic gene for the expression of the porcine relaxin B chain which comprises the following sequence:

5' CAG TCG ACC AAC GAT TTC ATC -

3' GTC AGC TGG TTG CTA AAG TAG -

AAA GCT TGT GGA CGT GAG TTA -
TTT CGA ACA CCT GCA CTC AAT -

GTT CGT TTA TGG GTT GAG ATC -
CAA GCA AAT ACC CAA CTC TAG -

TGT GGC TCT GTA AGT TGG GGT -
ACA CCG AGA CAT TCA ACC CCA -

CGT ACC GCA 3'
GCA TGG CGT 5'

In a further embodiment of the present invention, the B gene contains, in addition to the coding sequence, a stop codon, an initiator methionine codon, restriction sites for Cla I and Bam HI in order to facilitate insertion into plasmids, such as pAT 153, and restriction sites for Sal I, Hind III, Bgl II and Rsa I, (see Figure 5 of the accompanying drawings). There are three alternative forms, each with a different 3' terminal region to code for the different C termini of the B chains.

In this embodiment, the present invention relates to a synthetic gene for the expression of the porcine relaxin B chain which comprises the following sequence:

5' CGATG CAG TCG ACC AAC GAT TTC -
3' TAC GTC AGC TGG TTG CTA AAG -

ATC AAA GCT TGT GGA CGT GAG -
TAG TTT CGA ACA CCT GCA CTC -

TTA GTT CGT TTA TGG GTT GAG -
AAT CAA GCA AAT ACC CAA CTC -

ATC TGT GGC TCT GTA AGT TGG -
TAG ACA CCG AGA CAT TCA ACC -

GGT CGT ACC GCA TAA G 3'
CCA GCA TGG CGT ATT CCTAG 5'

In another embodiment, the present invention relates to a synthetic gene for the expression of a porcine relaxin hybrid having a peptide link -Asp-Pro-Gly-Asp-Pro- between the B chain and the A chain which comprises the following sequence:

5' GAT CCC GGG GAT CCG 3'
3' CTA GGG CCC CTA GGC 5'.

The hybrid B-C-A gene, which contains only a part of the B gene from the Bgl II site, has restriction sites for Bgl II and Bam HI in order to facilitate insertion into a plasmid, for example, pWT 551, (see, for example, published European Patent Application No. 52002), containing the other part of the B gene, internal restriction sites for Rsa I, Bam HI, Xma I, Hinf I and EcoR2 and a stop codon, (see, for example, Figure 6 of the accompanying drawings wherein, for convenience, only the form for

the longest B chain is illustrated).

The genes were constructed by synthesising a number of oligonucleotide blocks.

The synthetic blocks selected are illustrated in Figures 7, 8 and 9 of the accompanying drawings. The blocks may be constructed using known synthesis techniques, (see, for example, Ito, et al, (1982), Nucl. Acids Res., 10, 1755-1769).

Once constructed, the oligomeric blocks of nucleotides were hybridised and ligated by known methods, (see, for example, Agarwal, et al, (1970), Nature, 227, 27-34).

Accordingly, a further embodiment of the present invention relates to a process for the production of such a synthetic gene which comprises the assembly and ligation of a number of oligonucleotide blocks.

The next stage was to produce appropriate plasmid recombinants and hence to transform suitable cells so that the desired expression may be obtained.

Accordingly, another embodiment of the present invention relates to a plasmid recombinant which comprises a plasmid vector having inserted therein at an insertion site such a synthetic gene, the plasmid vector reading in the correct phase for expression of the inserted gene and having a bacterial promoter upstream of and adjacent to the insertion site.

In a further embodiment, the present invention

relates to a process for the production of such a plasmid recombinant which comprises inserting such a synthetic gene into an appropriate plasmid vector.

Also, another embodiment of the present invention relates to a cell which has been transformed by having inserted therein such a synthetic gene or such a plasmid recombinant.

In a further embodiment, the present invention relates to a process for the production of such a cell which comprises inserting such a synthetic gene or such a plasmid recombinant into an appropriate cell.

More particularly, the A gene was ligated into the large fragment of pWT 571 following removal of the small restriction fragment produced by treatment with EcoRI and Hind II and cloned. The plasmid was converted into one in which the relaxin A gene was next to the ATG codon for expression by treating with EcoRI and digesting the single stranded ends with S1 nuclease to form flush ends which were religated. The B gene was first cloned into pAT 153 at the Cla I and Bam HI sites for replication. For expression, the B gene was excised, flush-ended at the Cla I end with S1 nuclease and inserted into the expression plasmid cut with Hind III and flush-ended with S1 nuclease. Partial digestion with S1 nuclease prior to ligation provides plasmids with slightly different ribosome binding sites. As an alternative, one method of cloning the B gene was to produce a synthetic B gene

having a flush 5' end (by making the oligomer CGATGCAGTC two bases shorter at the 5' end) which was ligated into Hind III/Bam HI digested pWT 551 with a flush-ended Hind III site. The hybrid B-C-A gene was cloned into pWT 551 already containing the expressing B gene following restriction with Bgl II and Bam HI.

Following expression of the A and B genes after insertion into the above-mentioned plasmids and cloning in E. coli, (see, for example, Tacon, W.C.A., et al, (1980), Molec. Gen. Genet., 177, 427), the A and B chains may be extracted and purified from the bacteria and recombined to form active relaxin by first converting the terminal glutamine of the B chain to pyroglutamic acid by treatment at 100°C then forming the disulphide bridges. (See, for example, Katsoyannis, P.G., et al, (1967), Biochemistry, 6, 2642).

Following the expression of the B-C-A gene, the peptide product may be cleaved by hydrolysis at low pH, (see, for example, London, M., (1977), loc cit).

An alternative means of producing the B chain is as follows. The B gene may be ligated to the end of an expressing β-galactosidase gene and produced as a fused polypeptide with β-galactosidase. Cyanogen bromide cleavage at the methionine coded by the 5' end of the B gene releases a peptide identical to the B chain, (see, for example, Casabadan, M.J., (1980), J. Bact, 143, 971; and Goeddel, D.V., et al, (1979), Proc. Natl. Acad. Sci.

U.S.A., 76, 106-110).

Accordingly, another embodiment of the present invention relates to a process for the production of at least one chain of porcine relaxin which comprises culturing such a cell.

In a further embodiment, the present invention relates to a process for the production of porcine relaxin which comprises linking relaxin A and B chains produced by such a process via disulphide bridges.

In a further embodiment, the present invention relates to a process for the production of porcine relaxin which comprises cleaving a porcine relaxin hybrid produced by such a process at low pH.

The following illustrates the present invention:

Synthesis of the tridecanucleotide TpCpCpGpTpApApApGpApCpApT:

The completely protected tridecanucleotide was built up as illustrated in Figure 10 of the accompanying drawings wherein, for convenience, protecting groups are not shown. The reactions indicated by arrows in accompanying Figure 10 were carried out by the following procedures.

Commercially available, chloromethylated, 1% crosslinked polystyrene (0.32 meq Cl/g) was converted to the amino-methyl form (Ⓟ) by known methods, (see, for example, Mitchell, et al, (1976), Tetrahedron Letters No. 42, 3795-3798). 5'-dimethoxytrityl thymidine 3'-O-succinate was coupled to the polystyrene resin via an amide linkage by known methods, (see, for example, Miyoshi, et al, (1980), Nucl. Acids Res., 8, 5507-5517).

The preparation of dinucleotides was carried out by the following procedure exemplified in the synthesis of

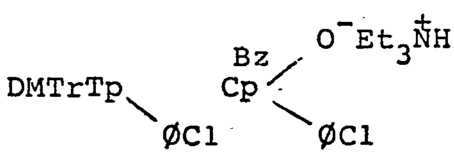

(VI)

as illustrated in Figure 11 of the accompanying drawings.

3 mmoles of

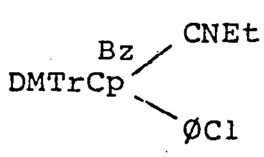

(I)

was reacted with 100 ml of 2% (w/v) benzene sulphonic acid in dichloromethane/methanol (7:3 v/v) for 5 minutes at 0°C. After extraction with 5% aqueous $NaHCO_3$ solution, the dichloromethane layer was dried and evaporated under

vacuum to an oil. The product (II) was purified by chromatography on silica using a gradient elution of dichloromethane to 10% MeOH/dichloromethane.

3.15 mmoles of

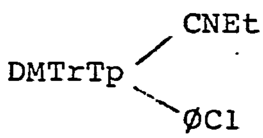

(III)

was reacted with $Et_3N$/pyridine (1:1 v/v) for 3 hours at room temperature and evaporated under vacuum to an oil. The product,

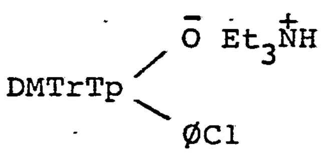

(IV)

was condensed with

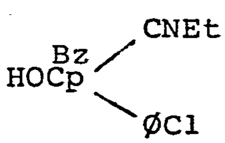

(II)

in anhydrous pyridine in the presence of 8 mmoles of mesitylene sulphonyl nitro triazole (MSNT) for 1 hour. The reaction was quenched with 5% (w/v) aqueous sodium bicarbonate and extracted with dichloromethane. The extract was evaporated under vacuum to an oil and the product purified by chromatography on silica using a gradient of dichloromethane to 10% MeOH/dichloromethane. The product (V) was treated with $Et_3N$/pyridine (1:1 v/v) to remove the cyanoethyl protecting group and evaporated under vacuum to a solid foam (VI) in an overall yield of 75%.

The solid was stored at -20°C ready for use in oligonucleotide synthesis.

100 mg of the polymer support with DMTrT attached was treated sequentially with:

(1) 2% bovine serum albumin (BSA) in dichloromethane/butanol (7:3) for 5 minutes

(2) dichloromethane/butanol (7:3) x 5

(3) dry pyridine x 5

(4) 100 mg dinucleotide and 100 mg MSNT in 0.5 ml dry pyridine for 45 minutes

(5) dry pyridine x 2

(6) 10% acetic anhydride/pyridine for 10 minutes

(7) dry pyridine x 2

(8) dichloromethane/butanol (7:3) x 5

This cycle was repeated with the appropriate dinucleotide until the oligonucleotide chain was complete. The coupling efficiency at each stage was determined by spectrophotometric assay of the liberated dimethoxytrityl alcohol at 504 nm.

At the completion of the synthesis, the oligonucleotide was removed from the polymer support and all protecting groups removed by sequential treatment with 0.5 M 1,1,3,3-tetramethyl guanidinium p-nitrobenzaldoximate for 48 hours at room temperature, concentrated ammonia for 5 hours at 70°C and 80% acetic acid for 30 minutes at room temperature. The deprotected oligonucleotides were purified by ion-exchange HPLC.

Hybridisation and ligation of oligomeric nucleotide blocks:

The series of steps in the construction of the B chain duplex is illustrated in Figure 12 of the accompanying drawings.

Oligonucleotides were phosphorylated with 0.25 mM ATP and 500 µCi γ-[$^{32}$P]-ATP per ml (50 mM Tris-HCl (tris(hydroxymethyl)aminomethane hydrochloride) pH 9.4, 10 mM $MgCl_2$, 5 mM dithiothreitol (DTT)) containing from 100 to 150 µg of oligonucleotide per ml and 125 units (1 unit is the amount that catalyses the production of 1 nmole of acid-insoluble $^{32}$P after incubation for 30 minutes at 37°C according to Richardson, C.C., (1972), Progress in Nucleic Acids Research, 2, 815) per ml of $T_4$ polynucleotide kinase (EC 2.7.1.78). The phosphorylated oligonucleotides were precipitated with ethanol and purified on a 20% (w/v) polyacrylamide gel under denaturing conditions. After elution from the gel, the recovery was determined by Cerenkov counting. In the ligation of, for example, blocks 11 and 13:

12.5 pmoles 5'-OH relaxin 11, 10 pmoles 5'-$^{32}$P-relaxin 13 and 10 pmoles 5'-OH relaxin 12 in 50 µl of water were annealed by heating to 100°C, followed by slow cooling to room temperature. The mixture was lyophilised and taken up in 20 µl of ligase solution (50 mM Tris-HCl pH 7.6, 10 mM $MgCl_2$, 20 mM DTT and 250 µm ATP) containing 200 units (1 unit is the amount that catalyses the conversion of 1 nmole of $^{32}$PPi into (α/β $^{32}$P)-ATP in 20 minutes at 37°C according to Weiss, B., et al, (1968), J. Biol. Chem., 243, 4543) of $T_4$ DNA ligase (EC 6.5.1.1). After 18 hours at 22°C, the reaction was terminated by the addition of 20 µl of 4 M ammonium acetate, 10 µg of tRNA and 3 volumes of ethanol. After precipitation, the product was purified on a 15% polyacrylamide gel under denaturing conditions.

Cloning of A gene in pWT 571:

pWT 571 was cleaved with restriction endonucleases EcoRI (EC 3.1.4.32) and Hind III (EC 3.1.23.21) according to the manufacturers instructions (New England Biolabs, Inc. Beverley MD, USA) and the 3628 bp fragment purified by agarose gel electrophoresis (1% low gelling temperature agarose (Bethesda Research Laboratories, Inc. Gaithersburg, MD, USA) in 0.09 M Tris-HCl pH 8.3, 2.5 mM sodium EDTA, 0.089 M boric acid). The large band was removed by melting the slice of agarose at 65°C, extracting with phenol/$CHCl_3$ (1:1 v/v) and precipitating with ethanol. The A gene was ligated to the plasmid in a 20 µl mixture containing 10 µg of A chain gene, 20 µg of the EcoRI/Hind III large fragment of pWT 571, 50 mM Tris-HCl pH 7.8, 10 mM $MgCl_2$, 1 mM ATP, 20 mM DTT, 200 units of T4 DNA ligase (New England Biolabs, Inc) incubated for 18 hours at 15°C. The ligated product was transformed into E coli K12 WT 217 (ara D139, Δ(ara, leu) 7697, Δ lac X74, gal $V^-$, gal $k^-$, $hsr^-$, $hsm^+$, str A, rec A56), using known methods, (see, for example, Cohen, et al, (1972), Proc. Natl. Acad. Sci. USA, 69, 2110-2114) and transformants resistant to 100 µg/ml ampicillin selected. Several transformants were further analysed by restriction enzyme cleavage of plasmid DNA, one being selected which was shown to contain a 70-80 bp insert on cleavage with EcoRI and Hind III and which contained, apparently within this same insert, a restriction site for Hinf I which is known to be within the A gene.

This plasmid, designated pRA 4, was then modified in such a way as to enable the A gene to be expressed. The plasmid was cut with EcoRI. The single stranded DNA "tail" at each end of the plasmid was removed to yield flush ends by treating with S1 nuclease (EC 3.1.4.21). 1.5 μg of EcoRI-cut plasmid pRA 4 was incubated with 7.5 units (1 unit is the amount that solubilises 10 μg nucleic acid in 10 minutes at 45°C according to Vogt, V.M., (1973), Eur. J. Biochem., 33, 192) of S1 nuclease for 0.5 hours at 15°C in 50 μl in 250 mM sodium chloride, 30 mM sodium acetate pH 4.5, 1 mM zinc sulphate. The product was phenol extracted, ethanol precipitated, ligated in a similar way to that described above, except that flush end ligation was favoured by using 800 units of T4 DNA ligase and a temperature of 25°C, and used to transform E coli K12 WT 217 as before. In this way, the ATG (initiator methionine codon) adjacent to the EcoRI site in the plasmid pWT 571 is placed next to the first codon of the A chain gene in the plasmid derived from pRA 4 (designated pRA 63 such that, under appropriate conditions, expression of the A chain gene occurs. Colonies containing the derived plasmid were selected for ampicillin resistance and the plasmids further selected by the insensitivity thereof to EcoRI treatment. The nucleotide sequence of the region just upstream of the old EcoRI site and including the entire A gene was determined by known methods, (see, for example, Maxam, A.M., and Gilbert, W., (1977),

Proc. Natl. Acad. Sci. U.S.A., 74, 560).

Expression of A gene:

Expression of relaxin A gene was induced by growth of cells in M9 medium containing, per litre, 6 g $Na_2HPO_4$, 3 g $KH_2PO_4$, 0.5 g NaCl, 1 g $NH_4Cl$, 0.25 g $MgSO_4.7H_2O$, 0.02 g $CaCl_2.6H_2O$, 5 g casamino acids lacking tryptophan (Difco), 5 g glucose, 1 mg thiamine and 100 mg carbenicillin (α-carboxy benzylpenicillin; Beecham), to an $A_{600nm}$ of 0.4. 3-β-indole acrylic acid was added to 20 mg/litre and incubated for 4 hours at 37°C. Under these conditions, maximal tryptophan promoter activity is known to occur and hence expression of the A chain, (see, for example, published G.B., Patent Application No. 2,068,970).

(Expression of the B and B-C-A genes was induced in a similar manner.)

Cloning of B gene in pWT 551:

Initially, the gene was inserted into pAT 153 and cloned. pAT 153 was restricted with restriction endonucleases Cla I and Bam HI (EC 3.1.23.6), (Bethesda Research Laboratories) and the resulting large fragment purified by electrophoresis on low gelling temperature agarose and extracted as previously described. The B gene was ligated to this plasmid fragment, the ligated product used to transform E coli K12 WT 217 and the transformants selected for ampicillin resistance as previously described. Several transformants were further analysed

by restriction enzyme cleavage of plasmid DNA to confirm the presence of a 100 bp insert containing restriction sites for Sal I (Hinc II), Hind III, Bgl II, Rsa I and bounded by sites for Cla I and Bam HI.

Next, the B gene was transferred from pAT 153 to an expressing site in pWT 551. pAT 153 containing the B gene was cut with restriction enzyme Cla I and treated with S1 nuclease as previously described, using 10 units of S1 nuclease with 10 µg plasmid DNA in order to remove the single stranded DNA at each end of the plasmid. In order to ensure flush ends, the plasmid was treated with DNA polymerase I (EC 2.7.7.7). 10 µg of Cla I, S1-treated plasmi was incubated at 15°C for 20 minutes with 20 units (1 unit is the amount that incorporates 10 nmoles of total nucleotides into an acid-precipitable fraction in 30 minutes at 37°C using poly-d(A-T) as primer according to Richardson, C.C., et al, (1964), J. Biol. Chem., 239, 222) of DNA polymerase I (large fragment - Bethesda Research Laboratories) in 100 mM Tris-HCl pH 7.4, 10 mM $MgCl_2$, 4 mM dithiothreitol and 0.5 mM of each of dATP, dCTP, dGTP, dTTP in 100 µl.

The plasmid was then cut with restriction enzyme Ava I and the resulting smaller fragment purified by agarose gel electrophoresis.

pWT 551 was cut with restriction enzyme Hind III, then treated with S1 nuclease and DNA polymerase I as described above in order to remove the single stranded

ends of the plasmid. The product was cut with Ava I and the larger fragment purified by agarose gel electrophoresis.

The two fragments thus produced were ligated under conditions which favour flush end ligation (described above) and used to transform E coli K12 WT 217 as before.

Recombinants containing pWT 551 with the inserted B chain were detected by the size of the intact plasmids, by the presence of the restriction sites within the B gene and the size of the B gene insert. Absolute confirmation of the presence of the definitive B gene, as well as the part of the plasmid flanking the 5' end of the gene, was obtained by nucleotide sequencing.

As mentioned above, an alternative approach involved producing a B gene with a flush 5' terminus from its constituent oligomers (oligomer ATGCAGTC being used in place of CGATGCAGTC in accompanying Figure 8) and to insert this into pWT 551 which has been treated with Hind III and Bam HI (the Hind III end having been flush-ended with S1 nuclease). Recombinants were screened as described above.

Since the S1 treatment was occasionally incomplete or excessive, recombinants containing the B gene were produced by these two methods which possessed from one to six bases in excess of or less than the desired plasmid in the region corresponding to the ribosome binding site next to the B gene. The expression of the B gene is affected by the sequence of this region.

Cloning of hybrid B-C-A gene in pWT 551:

pWT 551 containing the B gene at the Trp expression site was treated with restriction enzymes Bgl II and Bam HI and the large fragment purified by agarose gel electrophoresis. The B-C-A gene was ligated to this fragment and used to transform E coli K12 WT 217 as described above. Several transformants were analysed by restriction enzyme cleavage of plasmid DNA to confirm the presence of a 119 bp insert in the desired orientation containing restriction sites for Rsa I, Xma I, Hinf I, Bst NI and bounded by sites for Bgl II and Bam HI. The nucleotide sequence of a selected plasmid was determined to confirm the presence of the desired gene.

CLAIMS

1. A synthetic gene for the expression of the porcine relaxin A chain characterised in that it comprises the following sequence:

5' CGC ATG ACT CTG TCC GAG AAA TGT TGC CAG GTT GGT TGT-
3' GCG TAC TGA GAC AGG CTC TTT ACA ACG GTC CAA CCA ACA-

ATC CGT AAA GAC ATT GCT CGT CTG TGT 3'
TAG GCA TTT CTG TAA CGA GCA GAC ACA 5'

2. A synthetic gene as claimed in claim 1 comprising the following sequence:

5' A ATT CGC ATG ACT CTG TCC GAG AAA TGT TGC CAG GTT-
3' GCG TAC TGA GAC AGG CTC TTT ACA ACG GTC CAA-

GGT TGT ATC CGT AAA GAC ATT GCT CGT CTG TGT TA 3'
CCA ACA TAG GCA TTT CTG TAA CGA GCA GAC ACA ATT CGA 5'

3. A synthetic gene for the expression of the porcine relaxin B chain characterised in that it comprises the following sequence:

5' CAG TCG ACC AAC GAT TTC ATC AAA GCT TGT GGA CGT GAG-
3' GTC AGC TGG TTG CTA AAG TAG TTT CGA ACA CCT GCA CTC-

TTA GTT CGT TTA TGG GTT GAG ATC TGT GGC TCT GTA AGT TGG-
AAT CAA GCA AAT ACC CAA CTC TAG ACA CCG AGA CAT TCA ACC-

GGT CGT ACC GCA 3'
CCA GCA TGG CGT 5'

4. A synthetic gene as claimed in claim 3 comprising the following sequence:

5' CGATG CAG TCG ACC AAC GAT TTC ATC AAA GCT TGT GGA-
3' TAC GTC AGC TGG TTG CTA AAG TAG TTT CGA ACA CCT-

CGT GAG TTA GTT CGT TTA TGG GTT GAG ATC TGT GGC TCT-
GCA CTC AAT CAA GCA AAT ACC CAA CTC TAG ACA CCG AGA-

GTA AGT TGG GGT CGT ACC GCA TAA G 3'
CAT TCA ACC CCA GCA TGG CGT ATT CCTAG 5'

5. A synthetic gene for the expression of a porcine relaxin hybrid having a peptide link -Asp-Pro-Gly-Asp-Pro- between the B chain and the A chain characterised in that it comprises the following sequence:

5' GAT CCC GGG GAT CCG 3'
3' CTA GGG CCC CTA GGC 5'

6. A process for the production of a synthetic gene as claimed in any of claims 1 to 5 characterised in that it comprises the assembly and ligation of a number of oligonucleotide blocks.

7. A plasmid recombinant characterised in that it comprises a plasmid vector having inserted therein at an insertion site a synthetic gene as claimed in any of claims 1 to 5, the plasmid vector reading in the correct phase for

expression of the inserted gene and having a bacterial promoter upstream of and adjacent to the insertion site.

8. A process for the production of a plasmid recombinant as claimed in claim 7 characterised in that it comprises inserting a synthetic gene as claimed in any of claims 1 to 5 into an appropriate plasmid vector.

9. A cell which has been transformed by having inserted therein a synthetic gene as claimed in any of claims 1 to 5 or a plasmid recombinant as claimed in claim 7.

10. A process for the production of a cell as claimed in claim 9 characterised in that it comprises inserting a synthetic gene as claimed in any of claims 1 to 5 or a plasmid recombinant as claimed in claim 7 into an appropriate cell.

11. A process for the production of at least one chain of porcine relaxin characterised in that it comprises culturing a cell as claimed in claim 9.

12. A process for the production of porcine relaxin characterised in that it comprises linking relaxin A and

chains produced by a process as claimed in claim 11 via disulphide bridges.

13. A process for the production of porcine relaxin characterised in that it comprises cleaving a porcine relaxin hybrid produced by a process as claimed in claim 11 at low pH.

| | | | | | |
|---|---|---|---|---|---|
| | | | | 5 | |
| $NH_2$ — | Arg | Met | Thr | Leu | Ser - |
| | | | | 10 | |
| | Glu | Lys | Cys | Cys | Gln - |
| | | | | 15 | |
| | Val | Gly | Cys | Ile | Arg - |
| | | | | 20 | |
| | Lys | Asp | Ile | Ala | Arg - |
| | Leu | Cys | — | COOH | |

Fig. 1

| | | | | | |
|---|---|---|---|---|---|
| | | | | 5 | |
| $NH_2$ — | PGA | Ser | Thr | Asn | Asp - |
| | | | | 10 | |
| | Phe | Ile | Lys | Ala | Cys - |
| | | | | 15 | |
| | Gly | Arg | Glu | Leu | Val - |
| | | | | 20 | |
| | Arg | Leu | Trp | Val | Glu - |
| | | | | 25 | |
| | Ile | Cys | Gly | Ser | Val - |
| | | | | 30 | |
| | Ser | Trp | Gly | Arg | Thr - |
| | Ala — COOH | | | | |

Fig. 2

NH2 — PGA Ser Thr Asn Asp -
Phe Ile Lys Ala Cys -
Gly Arg Glu Leu Val -
Arg Leu Trp Val Glu -
Ile Cys Gly Ser Val -
Ser Trp Gly ¦ Arg ¦ Thr ¦ -
Ala - Asp - Pro - Gly - Asp - Pro -
Arg Met Thr Leu Ser -
Glu Lys Cys Cys Gln -
Val Gly Cys Ile Arg -
Lys Asp Ile Ala Arg -
Leu Cys — COOH

Fig.3

5' AATTCGC ATG ACT CTG TCC -
3' GCG TAC TGA GAC AGG -
EcoRI Hinf I
GAG AAA TGT TGC CAG -
CTC TTT ACA ACG GTC -
EcoR2
GTT GGT TGT ATC CGT -
CAA CCA ACA TAG GCA -
AAA GAC ATT GCT CGT -
TTT CTG TAA CGA GCA -
Stop
CTG TGT TA 3'
GAC ACA ATTCGA 5'
Hind III

Fig.4

5' CGATG CAG TCG ACC AAC GAT TTC -
3' TAC GTC AGC TGG TTG CTA AAG -
Cla I Sal I

ATC AAA GCT TGT GGA CGT GAG -
TAG TTT CGA ACA CCT GCA CTC -
Hind III

TTA GTT CGT TTA TGG GTT GAG -
AAT CAA GCA AAT ACC CAA CTC -

ATC TGT GGC TCT GTA AGT TGG -
TAG ACA CCG AGA CAT TCA ACC -
Bgl II

GGT CGT ACC GCA TAA G 3'
CCA GCA TGG CGT ATT CCTAG 5'
Rsa I Bam HI

GGT CGT TAA G
CCA GCA ATT CCTAG
Bam HI

GGT CGT ACC TAA G
CCA GCA TGG ATT CCTAG
Rsa I Bam HI

Fig.5

GATC TGT GGC TCT GTA AGT TGG GGT CGT ACC

ACA CCG AGA CAT TCA ACC CCA GCA TGG

Bgl II    Rsa I

GCG GAT CCC GGG GAT CCG CGC ATG ACT CTG

CGC CTA GGG CCC CTA GGC GCG TAC TGA GAC

Bam HI    Xma I    Bam HI    Hinf I

TCC GAG AAA TGT TGC CAG GTT GGT TGT ATC

AGG CTC TTT ACA ACG GTC CAA CCA ACA TAG

EcoR II

CGT AAA GAC ATT GCT CGT CTG TGT TAA G

GCA TTT CTG TAA CGA GCA GAC ACA ATT CCT AG

Bam HI

Fig.6

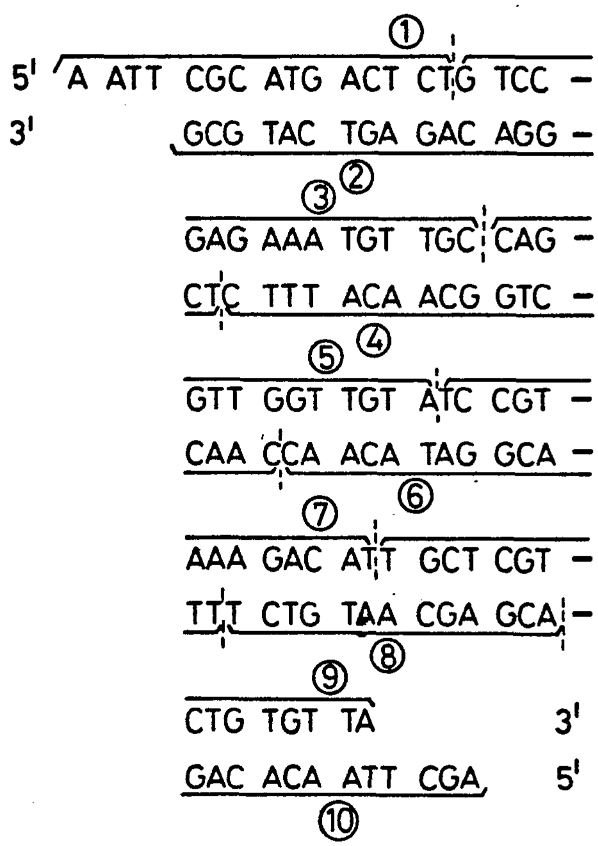
5' A ATT CGC ATG ACT CTG TCC –
3' GCG TAC TGA GAC AGG –
GAG AAA TGT TGC CAG –
CTC TTT ACA ACG GTC –
GTT GGT TGT ATC CGT –
CAA CCA ACA TAG GCA –
AAA GAC ATT GCT CGT –
TTT CTG TAA CGA GCA –
CTG TGT TA 3'
GAC ACA ATT CGA 5'
① ② ③ ④ ⑤ ⑥ ⑦ ⑧ ⑨ ⑩

Fig.7

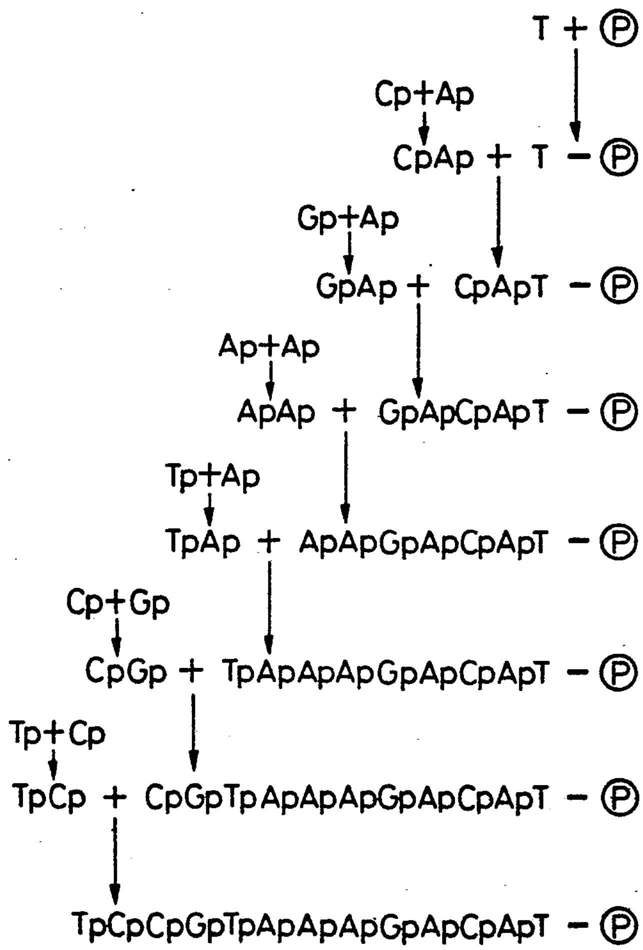
T + Ⓟ
Cp+Ap
CpAp + T – Ⓟ
Gp+Ap
GpAp + CpApT – Ⓟ
Ap+Ap
ApAp + GpApCpApT – Ⓟ
Tp+Ap
TpAp + ApApGpApCpApT – Ⓟ
Cp+Gp
CpGp + TpApApApGpApCpApT – Ⓟ
Tp+Cp
TpCp + CpGpTpApApApGpApCpApT – Ⓟ
TpCpCpGpTpApApApGpApCpApT – Ⓟ

Fig.10

```
       11                  13
5' CGATG CAG TCG ACC AAC GAT TTC -
3'      TAC GTC AGC TGG TTG CTA AAG -
               12

               15
        ATC AAA GCT TGT GGA CGT GAG -
        TAG TTT CGA ACA CCT GCA CTC -
        14                  16

        17
        TTA GTT CGT TTA TGG GTT GAG -
        AAT CAA GCA AAT ACC CAA CTC -
                     18

        19          21
        ATC TGT GGC TCT GTA AGT TGG -
        TAG ACA CCG AGA CAT TCA ACC -
               20                22

               23
        GGT CGT ACC GCA TAA G           3'
        CCA GCA TGG CGT ATT CCTAG       5'
                         24

            25
        GGT CGT TAA G          3'
            ATT CCTAG          5'
                 26

            27
        GGT CGT ACC TAA G              3'
            TGG ATT CCTAG              5'
                 28
```

Fig.8

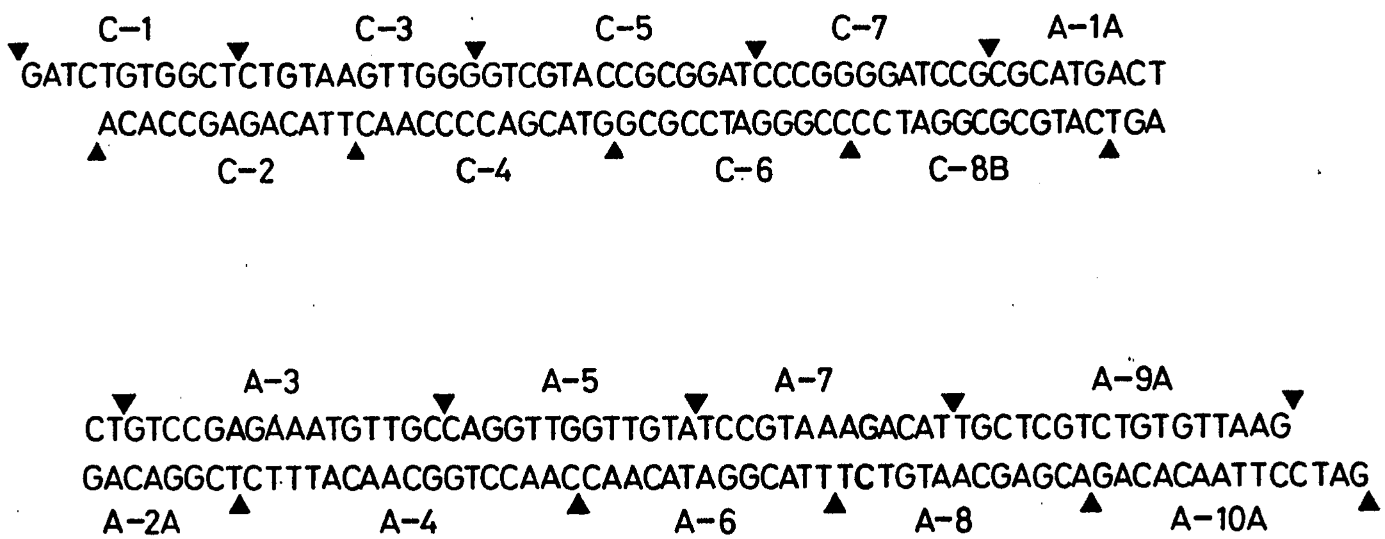
C-1
C-3
C-5
C-7
A-1A
GATCTGTGGCTCTGTAAGTTGGGGTCGTACCGCGGATCCCGGGGATCCGCGCATGACT
ACACCGAGACATTCAACCCCAGCATGGCGCCTAGGGCCCCTAGGCGCGTACTGA
C-2
C-4
C-6
C-8B
A-3
A-5
A-7
A-9A
CTGTCCGAGAAATGTTGCCAGGTTGGTTGTATCCGTAAAGACATTGCTCGTCTGTGTTAAG
GACAGGCTCTTTACAACGGTCCAACCAACATAGGCATTTCTGTAACGAGCAGACACAATTCCTAG
A-2A
A-4
A-6
A-8
A-10A

Fig.9

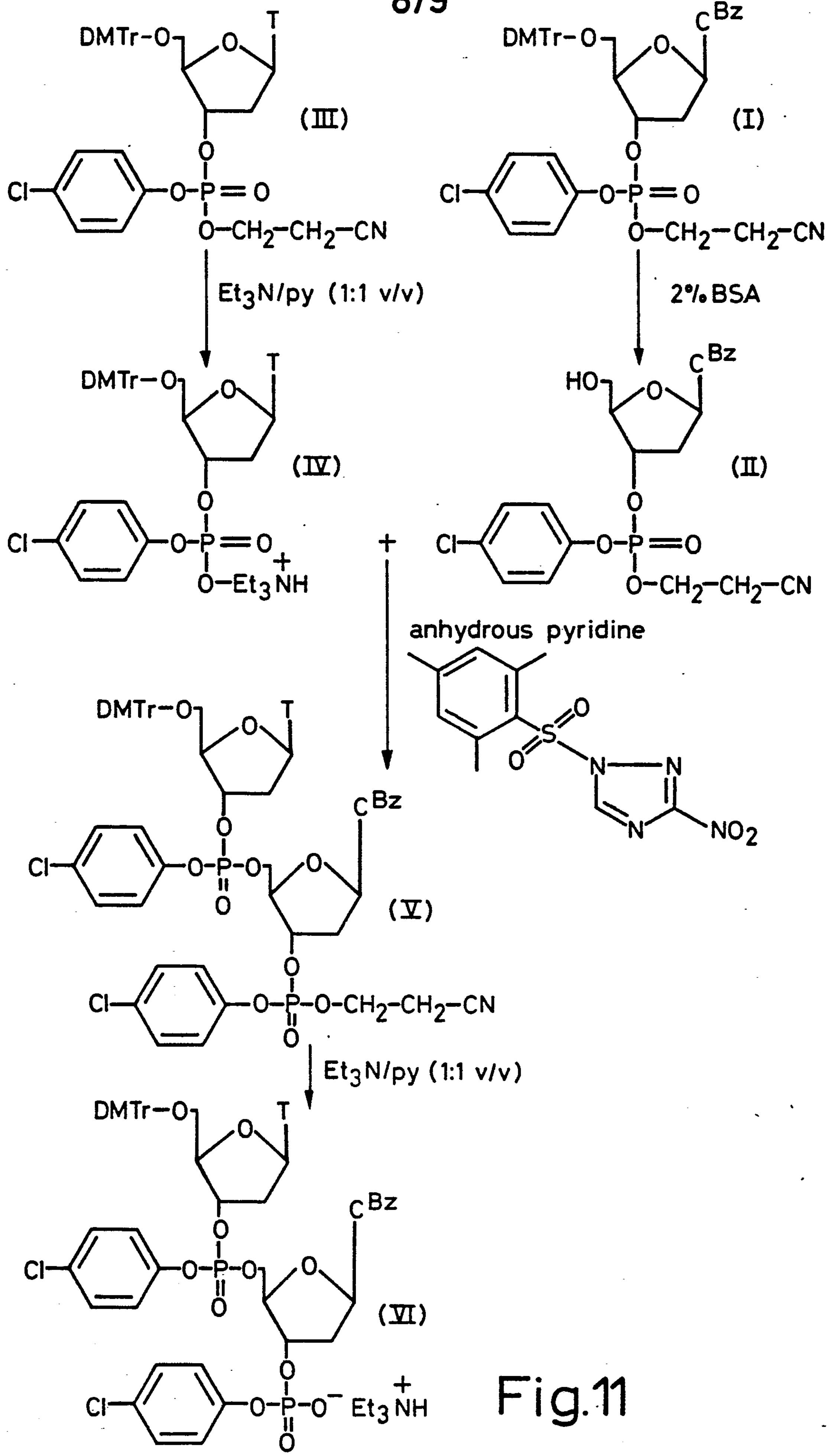
(III)
(I)
$Et_3N$/py (1:1 v/v)
2% BSA
(IV)
(II)
+
anhydrous pyridine
(V)
$Et_3N$/py (1:1 v/v)
(VI)

Fig.11

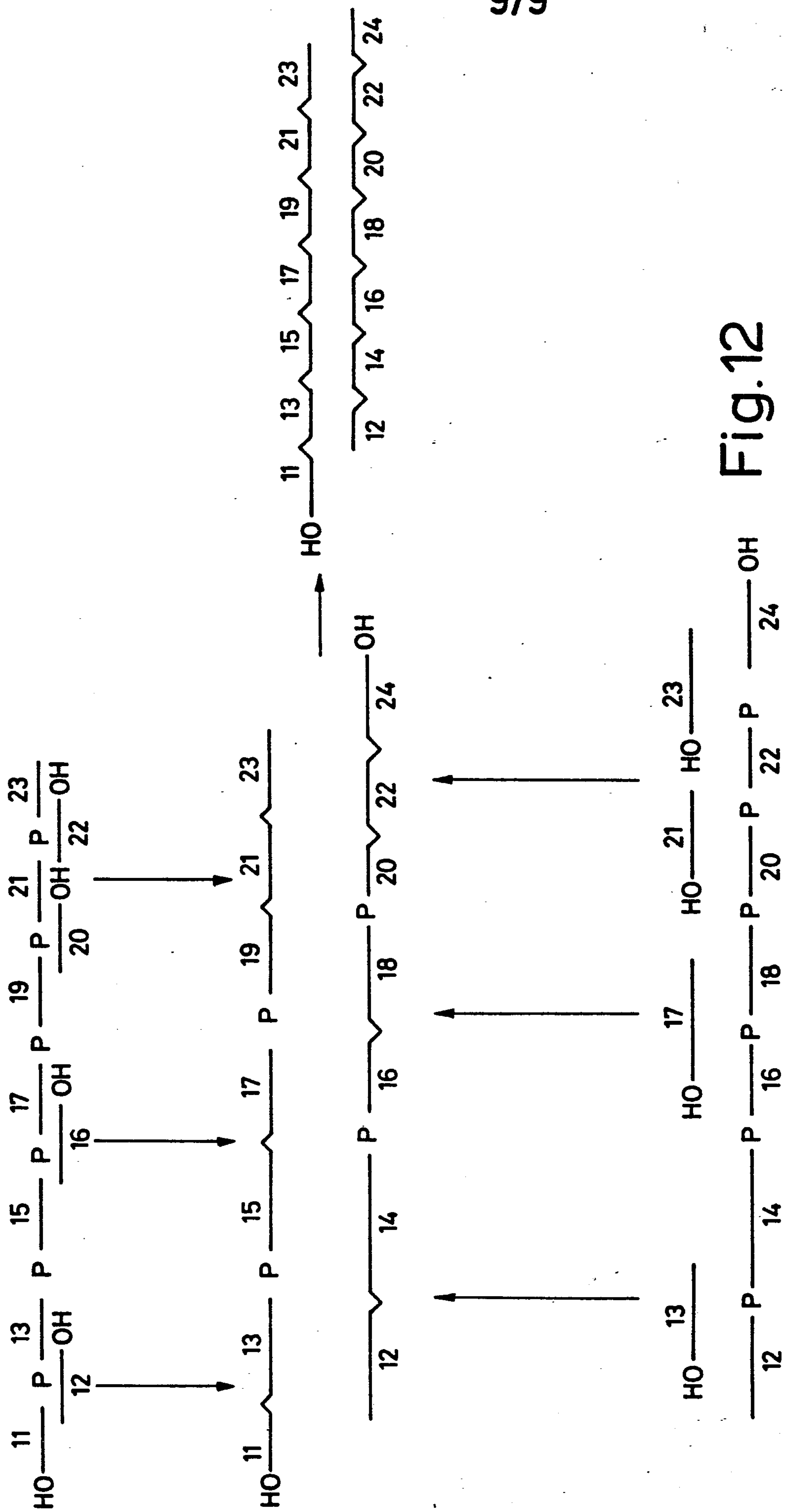

Fig.12